# EUROPEAN PATENT APPLICATION

(11) **EP 2 302 104 A1**
(43) Date of publication of application: **30.03.2011**
(21) Application number: 08787617.3
(22) Date of filing: 19.06.2008
(51) Int. Cl.: C25D 5/54, C25D 5/56, C25D 13/06, C08F 32/06, C08F 132/06

(54) **METHOD FOR ELECTROCHEMICALLY COVERING AN INSULATING SUBSTRATE**

(71) Applicant: Fundacion Cidetec, 20009 San Sebastian (Guipuzcoa) (ES)
(72) Inventor: CHÁVEZ AMADO, Esther, E-20009 San Sebastian (ES); DIEZ SILANES, Jose, Antonio, E-20009 San Sebastian (ES); MONTES PÉREZ, Sarah, E-20009 San Sebastian (ES); OCHOTECO VAQUERO, Estíbaliz, E-20009 San Sebastian (ES); POMPOSO ALONSO, José, Adolfo, E-20009 San Sebastian (ES)
(74) Representative: Carpintero Lopez, Francisco
(86) International application number: PCT/ES2008/000436
(87) International publication number: WO 2009/153361

(57) **Abstract**

The present invention relates to a process for obtaining a metal, ceramic or composite coating on the surface of a non-conductive material such as plastic, ceramic or wood which comprises: A) preparing a polypyrrole dispersion in aqueous base paint or in an acid type water-soluble pure resin; B) diluting the dispersion resulting from the previous stage with an alcohol in a factor of 1.5; C) applying the dispersion of the conductive polymer resulting from stage B) on the surface to be coated and drying same; and D) obtaining the metal, ceramic or composite coating by means of an electrolytic process or an electrophoretic deposition.

## Description

### Field of the Invention

The present invention relates to obtaining a ceramic, metal or composite coating on a non-conductive substrate, such as a plastic or ceramic substrate. More specifically the invention relates to a new process for obtaining a ceramic, metal or composite coating which comprises generating, from a colloidal polypyrrole dispersion, a conductive layer on the non-conductive material and then carrying out an electrochemical process.

### Background of the Invention

Various techniques for metallizing and depositing ceramic layers on insulating materials such as plastic or ceramic are currently known in the state of the art. The processes are generally based on the impregnation of the surface of the insulating material with a material allowing the passage of current in the processes for the electrodeposition of metals or for the electrophoretic deposition of ceramic particles. In this sense, there are various technologies in the state of the art for transforming an insulating surface into a conductive surface.

The metallization of a non-conductive material such as a ceramic or a plastic can be performed by means of different processes. The most used conventional process comprises the following stages for generating a conductive layer on the surface of the insulating material: (i) mordanting or conditioning; (ii) neutralization (iii) activation or channeling and (iv) chemical deposition. In the latter stage, a thin layer of the metal, which in most cases does not reach one micron, is formed on the surface of the part, which becomes conductive. Occasionally, highly toxic Cr(VI) is used. This process generally has the serious drawback that very low thickness of this metal film are obtained, which makes it necessary to reinforce it by electrolytically applying a second layer in order for it to have a suitable metal section for the passage of the current in the points of contact with the frame. The second layer is obtained, for example, by applying a flash of electrolytic nickel and, thereafter, the successive metal layers of copper-nickel-chromium are applied.

Likewise, there are other alternative processes for metallizing plastic, among the method for etching non-conductive surfaces, such as ABS, with a solution of halogenides and/or nitrates described in EP 1785507 should be mentioned. Other processes use solutions containing metal salts such as silver, palladium, molybdenum for example. JP2005146330 thus describes a surface treatment method for a non-conductive material such as ABS which uses the activation with Ag salts. JP62139896 describes the use of polypyrrole in the process for metallizing a plastic, but it is mixed with the molten plastic itself. In another process, the non-conductive surface is reacted with an alkaline permanganate solution to form a manganese dioxide chemically fixed by adsorption; the surface is then contacted with an aqueous acid solution containing 0.1 to 200 g/I of pyrrole or the derivative oligomers thereof, an electrically conductive layer being formed; and finally an electric current suitable for depositing the metal is applied. According to another process an oxidizer solution is applied to activate the non-conductive surface, followed by a treatment in a solution containing a heterocyclic monomer such as pyrrole, thiophene, furan or the derivatives thereof for example. The surface is then metallized by means of a galvanostatic technique.

On the other hand, various processes for obtaining a metal coating on a ceramic surface are likewise known. Thus, for example, WO 0104072 describes a process which comprises impregnating a ceramic surface with a solution containing metal particles, such as a paint applied by spraying. The conductive surface obtained thus allows the electrodeposition of a metal coating. US 4289829 describes a ("moly-manganese") process for metallizing a ceramic substrate which comprises applying a thin layer of paint containing molybdenum on the ceramic surface and sintering the part under a reduced atmosphere. US 3998602 describes a process for metallizing a non-conductive material which uses polymerizable monomers in the presence of small amounts of silver ions.

The mentioned processes generally have various drawbacks. Thus, strong oxidizing substances such as Cr (VI), palladium salts, silver salts, as well as the mixtures of substances which are highly toxic for the environment are used, and furthermore the number of stages of the processes is high, which lengthens the final process and makes it more expensive.

With respect to obtaining ceramic coatings on a non-conductive substrate, the existing techniques have experienced a great development since the 1970s decade. In fact, there is currently a huge interest in obtaining ceramic coatings with improved and combined properties such as hardness, coefficient of friction, tenacity, electrical, optical, magnetic properties and corrosion resistance of the substrates for electronic, medical and mechanical applications. Various processes which use the electrophoretic deposition and in which the substrate must therefore be conductive are known in the state of the art.

In this sense, a process is known in which the coating is injected by applying a current by means of a needle, also using small conductive plates which are directly placed and spaced on the working area.

JP493970 describes a process for depositing ceramic particles which consists of formulating the conductive polymer in the presence of ceramic particles and thus forming a conductive layer thereon. WO 200711010 describes obtaining metal and ceramic parts in a varied manner by electrophoretic deposition. In this process, a metal or ceramic powder suspension is injected into the substrate through a hollow needle. The deposition of material occurs by applying a voltage between the suspension and the substrate.

In any case, the state-of-the-art processes in which electrophoretic deposition is used are always performed on the metal substrates and, furthermore, they later need a machining process.

Other state-of-the-art processes describe the manufacture of dental prostheses of an exclusively ceramic nature, which are therefore biocompatible, have a high resistance to masticatory forces and much higher esthetic values than those of ceramometal prostheses. Currently, in the manufacture of these ceramic dental prostheses, the application of the decorative ceramic layers giving the final finish to a ceramic prostheses (final enamel) is performed manually, by the application with a paintbrush, which slows down the process and makes it more expensive. Furthermore, the manual manufacture gives rise to the problems of dimensional inaccuracy and lack of homogeneity in the volume of applied material, the subsequent correction and dimensional adjustment prior to the implantation thereof being necessary.

Application US 2007029199 describes the formation of ceramic substrates for dental applications by means of electrophoretic deposition. Metal or ceramic substrates are used to which a layer of silver paint and a hygroscopic layer have been applied to prevent bubble formation. The state-of-the-art electrophoretic deposition processes are generally applied on metal substrates and may need an additional machining stage.

From what has been set forth above it is inferred that there is still a need in the state of the art to provide an alternative process for obtaining ceramic, metal or composite coatings on a non-conductive substrate which overcomes at least part of the aforementioned drawbacks.

In this sense, the inventors of the present invention have discovered a new process by means of which it is possible to obtain a ceramic, metal or composite coating on an insulating substrate, based on the generation of a conductive layer from a colloidal polypyrrole dispersion on the surface of the insulating substrate, and the use of said conductive layer in obtaining one of said coatings electrochemically.

### Description of the Invention

In one aspect, the invention relates to a process for obtaining a ceramic, metal or composite coating on an insulating substrate which comprises the following stages:
A) preparing a polypyrrole dispersion in aqueous base paint or in an acid type water-soluble pure resin;
B) diluting the dispersion resulting from stage A) with an alcohol in a factor of 1.5;
C) applying the dispersion resulting from stage B) on the surface to be coated and drying same; and
D) obtaining the metal, ceramic or composite coating by means of an electrolytic or electrophoretic deposition process.

The process, hereinafter the process of the invention, can be applied on any type of non-conductive substrate of a different nature, such as for example plastic, ceramic, wood, among others.

The aqueous base paint useful to put the invention into practice can be a commercial outdoor acrylic water-based paint, and the acid type water-soluble pure resin can be a acrylic resin in an aqueous base with a pH comprised between 2 and 5.

Stage A) of the process of the invention comprises obtaining in the first place a polypyrrole dispersion in aqueous medium by pyrrole polymerization at room temperature. The resulting dispersion is taken to dryness either by lyophilization or in a rotary evaporator for the complete evaporation of water. The dry material obtained is redispersed in a commercial aqueous base paint or in an acid type water-soluble pure resin, maintaining the stirring. In a particular embodiment, the concentration of this dispersion is comprised between 0.05 and 2.5 gram of polypyrrole/g of paint or resin.

A particular embodiment of the process of the invention starts from the monomer pyrrole, at a concentration comprised between 1 x 10⁻⁴ and 1 x 10⁻¹ g/ml in aqueous medium, and an anionic dispersant of the type of polystyrene sulfonate, poly(vinyl sulfonate), etc. with a molecular weight comprised between 10,000 and 1,000,000 g/mol. This mixture is submerged in a bath at a temperature below 0ºC and stirred by ultrasound. Once it is cooled, the oxidizer previously dissolved in water, of the type of ammonium persulfate, iron trichloride, etc., is added, following a process well known in the state of the art. The polymerization reaction is usually carried out at room temperature with mechanical stirring at 300 r.p.m, for a time usually comprised between half an hour and 7 hours. Once the reaction has ended, the stirring is stopped and the polypyrrole dispersion is removed from the bath.

In stage B) of the process of the invention, the polypyrrole dispersion in paint or resin is then diluted with an alcohol in a factor of 1.5 until achieving a homogenous polypyrrole dispersion, ready to be used. In a particular embodiment, the alcohol is isopropanol.

The final steps of drying, redispersing and diluting are necessary for the final paint or resin to not coagulate, be perfectly homogenous, have a lower viscosity, and not be as diluted as if the aqueous paint and the polypyrrole dispersion were directly mixed without having been previously taken to dryness.

Finally, the electrical conductivity of the conductive polymer can be optionally determined by means of any conventional method.

Stage C) of the process of the invention refers to the application of the dispersion of the conductive polymer which is performed by the immersion of the part to be coated, painting or spraying. In a particular embodiment, the application is performed by the immersion of the part in the paint containing the colloidal polypyrrole and subsequent drying in a temperature range comprised between 10 and 80ºC, preferably room temperature. Finally, the part is washed with distilled water.

Stage D) of the process consists of obtaining the final coating on the surface to which the conductive polymer has been applied. The various processes for obtaining each type of coating are described below:
D-1) Obtaining a metal coating by means of an electrolytic process: It is necessary to have a simple cell containing the electrolyte and the counter electrodes (anode and cathode) to obtain coatings of this type. Any conventional electrolyte or electrodeposition bath (silver, copper, nickel, gold, etc.) which is commonly used in conventional industrialized processes can be used to perform this galvanic process. The current used in the electrodeposition process can be direct current or by means of current pulses (depending on the properties to be obtained in the final coating).
D-2) Obtaining a composite coating by means of a electrolytic process: It is necessary to have a simple cell containing the electrolyte and the counter electrodes (anode and cathode) to obtain coatings of this type. Any conventional electrolyte or electrodeposition bath of those which are commonly used in industrialized processes can be used as an electrolyte. The electrolyte comprises at least one metal salt and at least one ceramic particle. Examples of metal salts are salts of metals such as silver, copper, nickel, gold, among others. The ceramic particles can be of SiC, Al₂O₃, SiO₂, ZrO₂, etc. The current used in the electrodeposition process can be direct current or by means of current pulses (depending on the properties to be obtained in the final coating).
D-3) Obtaining a ceramic coating by means of an electrophoretic deposition process. This process is performed in the following steps:
   Preparing the suspension of ceramic particles in an aqueous medium to which the ceramic particles are added, at a concentration comprised between 1-60 % by weight, preferably between 4-30% by weight with respect to the total weight of the suspension. The types of ceramic particles that can be used in the present invention are, among others, oxides such as SiO₂, ZrO₂, Al₂O₃, TiO_{2;} carbides, such as SiC, nitrides, such as NTi; and dental ceramic particles such as Base Dentine, Enamel, Dentine 3M-2, Effect Peral, Effect Croma, which can be commercially acquired, for example, of the VITA Zahnfabrik trademark, as well as other ceramics which are conventionally used for generating esthetic layers on dental ceramic substrates.

A series of dispersant, binder and/or plasticizer additives are then added, always in a percentage which can vary between 0.1-2% by weight with respect to the total weight of the suspension, for the purpose of stabilizing the suspension. The additives used are: amines, polymethacrylates, pyrophosphates, citric acid, hydrochloric acid, methyl cellulose, etc. To homogenize and generate an electric charge density in the surface of the ceramic particles, they are stirred by means of ultrasound for a time ranging between 1 to 60 minutes.

The ceramic coating is obtained by means of an electrophoretic deposition, for which a simple cell containing the previously prepared suspension and the counter electrodes (anode and cathode) is used. Potentials varying between 20 and 400 V are applied for a time ranging between 1 and 40 minutes for the formation of the ceramic coating. For the consolidation of the coating, the latter is subjected to a sintering process, applying temperatures comprised between 400ºC and 1500ºC, preferably at temperatures above 800ºC.

The process of the present invention has a number of advantages in comparison with the processes of the art. It should be emphasized that the use of metal particles such as silver, of baths based on strong oxidizers such as Cr(VI), substances which are highly toxic for the environment, is eliminated. Furthermore, the number of stages necessary for generating a conductive layer on an insulating surface is reduced, with respect to conventional processes for metallizing plastic, to the application of the conductive polymer in an aqueous base and to the drying thereof. Therefore, the process of the invention achieves, in a single stage, transforming an insulating surface into a conductive surface using a colloidal polypyrrole dispersion in an aqueous paint or resin, which furthermore considerably reduces the cost and time of the process.

On the other hand, the process of the invention has the additional advantage that it allows obtaining the final ceramic layers in a few minutes, using to that end an electrophoretic deposition process. This process can be automated, further simplifying the process. The process of the invention therefore allows, in a particular embodiment, obtaining biodegradable and ceramic dental prostheses quickly and with a high volumetric accuracy. The process is performed on final ceramic teeth; the time of application of these decorative layers of ceramic is reduced from one day to a few minutes. The global process is therefore simplified and the manufacturing time of the prostheses is significantly reduced.

Illustrative examples of the invention are set forth below to better understand the invention and in no case should they be considered as a limitation of the scope thereof.

### Examples

### Example 1. Preparation of a ceramic coating for a dental prosthesis.

Aqueous polypyrrole dispersion was prepared in the first place. To that end, a volume of pyrrole of 0.5-10 ml was added to an aqueous solution containing 1-5% polystyrene in water. The resulting mixture was submerged in a bath at a temperature equal to 0ºC and stirred by ultrasound. Once cooled, ammonium persulfate previously dissolved at a concentration of 6.84 g in 50 ml of water was added. After two hours of reaction, the stirring was stopped and the polypyrrole dispersion was removed from the bath. The resulting dispersion was lyophilized. Then, 100 ml of commercial outdoor acrylic water-based paint (BRUGUER) were taken, to which 15 g of lyophilized polypyrrole were added, maintaining the stirring. To reduce the viscosity of the mixture, 25 ml of isopropanol were used as a diluent. A homogenous polypyrrole dispersion in the ready paint was obtained, which was applied by means of the immersion of a rectangular ABS (acrylonitrile-butadiene-styrene copolymer) part obtained by injection.

An aqueous suspension of Enamel ceramic particles at a concentration equal to 3% by weight was then prepared. The following additives were then added: 10% polymethacrylate and 5% ethanol.

To homogenize and generate an electric charge density in the surface of the ceramic particles, they were stirred by means of ultrasound for 15 minutes.

The part was then submerged in a simple cell containing the suspension of ceramic particles. The part with the conductive polymer on its surface was connected to one of the electrodes, and closing the electric circuit, a metal counter electrode of stainless steel was used. A potential of 100 V was applied for a time equal to 10 minutes for the formation of the ceramic coating.

For the consolidation of the coating, the part was subjected to a sintering process which comprised subjecting the part to a pre-drying temperature of 500ºC for 6 minutes, applying a heating gradient of 55ºC/min until reaching the cooking temperature of 910ºC and maintaining this temperature for 1 minute.

A completely ceramic dental prosthesis was obtained.

### Example 2 Preparation of a metal coating on plastics

An aqueous polypyrrole dispersion was prepared in the first place as in Example 1. The dispersion was applied by means of immersion to the ABS part.

The resulting part was subsequently introduced in a simple cell containing the counter electrodes (anode and cathode) and a conventional electrolyte of acid copper as an electrolyte or electrodeposition bath. The current used in the electrodeposition process was 3A/dm².

An ABS part with homogenous and shiny metal appearance was obtained.

## Claims

1. Process for obtaining a metal, ceramic or composite coating on the surface of a non-conductive material which comprises the following stages:
A) preparing a polypyrrole dispersion in aqueous base paint or in an acid type water-soluble pure resin;
B) diluting the dispersion resulting from the previous stage with an alcohol in a factor of 1.5;
C) applying the dispersion resulting from stage B) on the surface to be coated and drying same; and
D) obtaining the metal, ceramic or composite coating by means of an electrolytic process or an electrophoretic deposition.

2. Process according to claim 1, wherein stage A) comprises:
A-1) obtaining polypyrrole in aqueous medium from pyrrole;
A-2) taking the resulting dispersion to dryness; and
A-3) redispersing the resulting dry material in aqueous base paint or in an acid type water-soluble pure resin until reaching a concentration comprised between 0.05 and 2.5 g of polypyrrole/g of paint or resin.

3. Process according to claim 2, wherein stage A-1) is performed starting from pyrrole in aqueous medium at a concentration comprised between 1 x 10⁻⁴ and 1 x 10⁻¹ g/ml; and an anionic dispersant with a molecular weight comprised between 10,000 and 1,000,000 g/mol; the mixture is submerged in a bath at a temperature below 0ºC and stirred by ultrasound; an oxidizer dissolved in water is added; the polymerization takes place at room temperature with mechanical stirring, for a time comprised between half an hour and 7 hours.

4. Process according to claim 2, wherein stage A-2) comprises lyophilizing or treating in a rotary evaporator the polypyrrole dispersion obtained in stage A-1).

5. Process according to claim 2, wherein stage A-3) comprises dispersing the dry material obtained in A-2) in a aqueous base paint or in an acid type water-soluble pure resin, reaching a concentration comprised between 0.05 and 2.5 g of polypyrrole/g of paint or resin.

6. Process according to the claim 1, wherein in stage B) the polypyrrole dispersion obtained in stage A) is then diluted with an alcohol in a factor of 1.5.

7. Process according to claim 1 or 2, wherein stage C) of applying the dispersion of polypyrrole to a part is performed by the immersion of said part, painting or spraying and subsequent drying.

8. Process according to claim 1, wherein stage D) comprises obtaining a metal coating on the surface to which the polypyrrole has been applied, by means of an electrolytic process in a simple cell containing an electrolyte with direct current or by means of current pulses.

9. Process according to claim 1, wherein stage D) comprises obtaining a composite coating on the surface to which the polypyrrole has been applied, by means of an electrolytic process in a simple cell containing an electrolyte which comprises at least one metal salt and at least one ceramic particle.

10. Process according to claim 1, wherein stage D) comprises obtaining a ceramic coating on the surface to which the polypyrrole has been applied, by means of an electrophoretic deposition in a simple cell containing a suspension of ceramic particles in an aqueous medium.

11. Process according to claim 10, wherein the concentration of ceramic particles in the suspension is comprised between 1-60% by weight with respect to the total weight of the suspension and between 0.1-2% by weight with respect to total weight of the suspension of additives.

12. Process according to claim 10, wherein a potential comprised between 20 and 400 V is applied for a time comprising between 1 and 40 minutes.

13. Process according to claim 10, wherein after the electrophoretic deposition the part obtained is sintered at a temperature comprised between 400ºC and 1500ºC.
